# EUROPÄISCHE PATENTSCHRIFT

(11) **EP 0 522 032 B1**
(45) Veröffentlichungstag und Bekanntmachung des Hinweises auf die Patenterteilung: **02.11.1994**
(21) Anmeldenummer: 91907269.4
(22) Anmeldetag: 27.03.1991
(51) Int. Cl.: C07D 307/935

(54) **NEUES VERFAHREN ZUR SYNTHESE VON PROPARGYLALKOHOLEN UND DESSEN ANWENDUNG ZUR HERSTELLUNG VON PROSTAGLANDINVORSTUFEN**
NEW PROCESS FOR SYNTHESIS OF PROPARGYL ALCOHOLS AND ITS USE TO PRODUCE PROSTAGLANDIN PRECURSEURS
NOUVEAU PROCEDE DE SYNTHESE D'ALCOOLS PROPARGYLIQUES ET SON UTILISATION DANS LA PRODUCTION DE PRECURSEURS DE PROSTAGLANDINE

(30) Priorität: 29.03.1990 DE 4010339
(43) Veröffentlichungstag der Anmeldung: 13.01.1993
(73) Patentinhaber: SCHERING AKTIENGESELLSCHAFT, 13342 Berlin (DE)
(72) Erfinder: KLAR, Ulrich, D-1000 Berlin 27 (DE)
(86) Internationale Anmeldenummer: EP9100579
(87) Internationale Veröffentlichungsnummer: WO9114684

(56) Entgegenhaltungen:
- EP-A- 0 284 547
- DE-A- 2 627 422
- Chemistry Letters, no. 9, Sept. 1984 The Chemical Society of Japan, Y. Torisawa et al., pages 1555-1556, see the wohole article, in particular table 1, example 7
- Liebigs Annalen der Chemie, no.1, January 1980, Verlag Chemie GmbH (Weinheim, DE) E.V. Dehmlow et al., pages 1-13, see the whole article, in particular page 5, paragraph 3-page 7, paragraph 1

## Beschreibung

Bei der Synthese von Prostaglandinanaloga mit einer Dreifachbindung in Position 13.14 (Prostan-Zählweise) wie sie beispielsweise in EP 0284547, DE 3610556, DE 3725031, DE 3428266, DE 3427797, US 4628110 beschrieben werden, wird die Dreifachbindung erst in einem relativ späten Synthesestadium mit vergleichsweise mäßigen Ausbeuten eingeführt. Es wurde nun überraschend gefunden, daß ausgehend von Verbindungen der allgemeinen Formel II die Dreifachbindung unter milden Bedingungen durch Eliminierung von Bromwasserstoff mit wasserfreiem Cäsiumacetat und 18-Krone-6 bereits in einem frühen Synthesestadium in sehr guten Ausbeuten generiert werden kann.
Das neue Verfahren stellt somit eine wertvolle Bereicherung der synthetischen Methodologie dar.
Die Erfindung betrifft somit ein Verfahren zur Herstellung von Prostaglandinvorstufen der Formel I 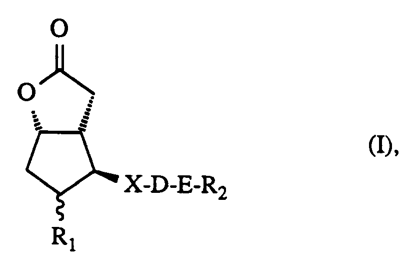
sowie deren Enantiomere
worin
- X: A-W oder W-A,
- A: eine ― C ≡ C ― Gruppe,
- W: eine Hydroxymethylengruppe, in der die OH-Gruppe durch Veretherung oder Veresterung funktionell abgewandelt sein kann,
- D: eine geradkettige oder verzweigtkettige Alkylengruppe mit jeweils 2-5 C-Atomen oder eine 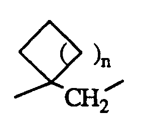 -Gruppe,
- n: 1 bis 3,
- E: eine ― C ≡ C ― Gruppe oder eine -CR₃=CR₄-Gruppe mit R₃ und R₄ jeweils in der Bedeutung eines Wasserstoffatomes oder einer C₁-C₄-Alkylgruppe,
- R₁: ein Wasserstoffatom oder eine Hydroxygruppe, die wie in W funktionell abgewandelt sein kann,
- R₂: eine geradkettige oder verzweigtkettige Alkylgruppe mit 1-7 C-Atomen ist, das dadurch gekennzeichnet ist, daß man Vinylbromide der Formel II 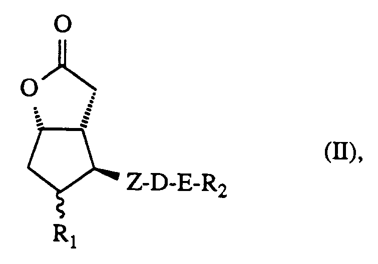
worin Z die Gruppe 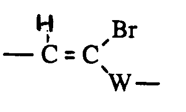
oder 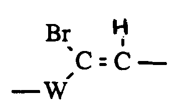
bedeutet und R₁, W, D, E und R₂ die oben angegebenen Bedeutungen haben und Hydroxygruppen in R₁ und W ungeschützt oder geschützt sein können durch einen gegebenenfalls substituierten Benzoylrest, eine C₁-C₆-Alkanoylgruppe, einen Tetrahydropyranylrest, einen Tetrahydrofuranylrest, eine Trialkylsilylgruppe oder eine Diphenylalkylsilylgruppe jeweils mit Alkyl in der Bedeutung eines C₁-C₄-Alkyls, mit wasserfreiem Cäsiumacetat in Gegenwart von 18-Krone-6 umsetzt.

### Beispiele

### Beispiel 1

(1S,5R,6S,7R)-7-Hydroxy-6-[(3S,4S)-3-hydroxy-4-methyl-non-1,6-diinyl]-2-oxabicyclo[3.3.0]octan-3-on: 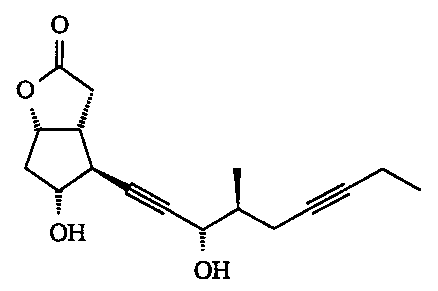
500 mg (1,35 mmol) (1S,5R,6S,7R)-7-Hydroxy-6-[(E)-(3S,4S)-2-brom-3-hydroxy-4-methyl-1-nonen-6-inyl]-2-oxabicyclo[3.3.0]octan-3-on (Darstellung siehe EP0284547) löst man in 40 ml wasserfreiem Toluol, versetzt mit 840 mg wasserfreiem Cäsiumacetat, 1,0 g 18-Krone-6 und erhitzt das farblose heterogene Gemisch 9 Stunden unter einer Atmosphäre aus trockenem Argon zum Rückfluß. Nach dem Erkalten engt man auf ca. 20 ml ein und reinigt das Gemisch durch Chromatographie an ca. 100 ml feinem Kieselgel mit Ethylacetat unter Druck. Isoliert werden 386 mg (1,33 mmol, 98%) der Titelverbindung als farbloses Öl.
IR (Film): 3700-3100, 2970, 2920, 2230, 1755, 1450, 1415, 1370, 1320, 1200, 1085, 1025, 980 und 900 cm⁻¹.

### Beispiel 2

(1S,5R,6S,7R)-7-Benzoyloxy-6-[(3R,4S)-3-hydroxy-4-methyl-non-1,6-diinyl]-2-oxabicyclo [3.3.0]octan-3-on: 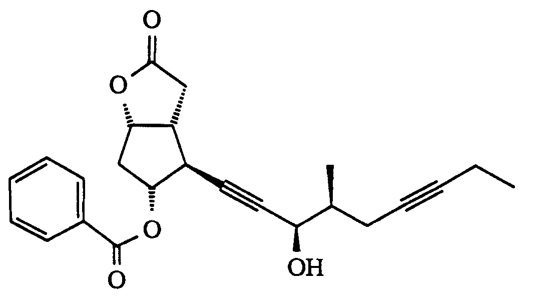
1,56 g (3,30 mmol) (1S,5R,6S,7R)-7-Benzoyloxy-6-[(E)-(3R,4S)-2-brom-3-hydroxy-4-methyl-1-nonen-6-inyl]-2-oxabicyclo[3.3.0]octan-3-on (Darstellung siehe EP0284547) setzt man in Analogie zu Beispiel 1 um und isoliert nach chromatographischer Reinigung 1,15 g (2,73 mmol, 83%) der Titelverbindung als farbloses Öl.
IR (Film): 3700-3100, 3060, 2970, 2920, 2870, 2230, 1770, 1715, 1600, 1450, 1365, 1315, 1270, 1180, 1110, 1070, 1030, 980, 900, 735 und 710 cm⁻¹.

### Beispiel 3

(1S,5R,6S,7R)-7-Benzoyloxy-6-[(3S,4S)-3-(4-nitrobenzoyloxy)-4-methyl-non-1,6-diinyl]-2-oxa-bicyclo[3.3.0]octan-3-on: 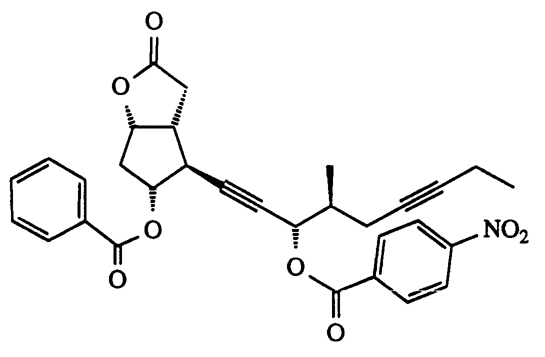
280 mg (1S,5R,6S,7R)-7-Benzoyloxy-6-[(E)-(3S,4S)-2-brom-3-(4-nitrobenzoyloxy)-4-methyl-1-nonen-6-inyl]-2-oxabicyclo[3.3.0]octan-3-on als kristallines Addukt mit 1,4 Äquivalenten 1.2-Bis-ethoxy-carbönylhydrazin (entspricht 321 µmol) setzt man in Analogie zu Beispiel 1 um und isoliert nach chromatographischer Reinigung 91 mg (167 µmol, 52%) der Titelverbindung als Feststoff neben 24% Ausgangsmaterial. IR (KBr): 3110, 3070, 2970, 2930, 2870, 2240, 1770, 1720, 1605, 1525, 1450, 1345, 1315, 1265, 1175, 1110, 1070, 965, 870, 850 und 710 cm⁻¹.

### Beispiel 4

(1S,5R,6R,7R)-7-Benzoyloxy-6-[(1R,4S oder 1S,4S)-1-hydroxy-4-methyl-non-2,6-diinyl]-2-oxabicyclo[3.3.0]octan-3-on: 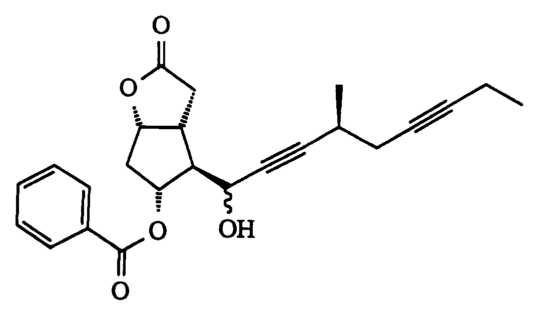
150 mg (315 µmol) (1S,5R,6R,7R)-7-Benzoyloxy-6-[(Z)-(1R,4S od. 1S,4S)-1-hydroxy-2-brom-4-methyl-non-2-en-6-inyl]-2-oxabicyclo[3.3.0]octan-3-on (polarer Alkohol aus Bsp. 4a) setzt man in Analogie zu Beispiel 1 um und isoliert nach chromatographischer Reinigung 81 mg (203 µmol, 65%) der Titelverbindung als farbloses Öl.
IR (Film): 3600-3100, 3060, 2970, 2965, 2935, 2230, 1770, 1710, 1600, 1450, 1375, 1315, 1270, 1175, 1110, 1070, 1045, 1025 und 710 cm⁻¹.

### Beispiel 4a

(1S,5R,6R,7R)-7-Benzoyloxy-6-[(Z)-(1R,4S)-1-hydroxy-2-brom-4-methyl-non-2-en-6-inyl]-2-oxa-bicyclo[3.3.0]octan-3-on und (1S,5R,6R,7R)-7-Benzoyloxy-6-[(Z)-(1S,4S)-1-hydroxy-2-brom-4-methyl-non-2-en-6-inyl]-2-oxabicyclo[3.3.0]octan-3-on:
17,6 g (37,1 mmol) (1S,5R,6S,7R)-7-Benzoyloxy-6-[(E)-(3R,4S)-2-brom-3-hydroxy-4-methyl-1-nonen-6-inyl]-2-oxabicyclo[3.3.0]octan-3-on (Darstellung siehe EP0284547) löst man in 260 ml Aceton,kühlt unter einer Atmosphäre aus trockenem Argon auf -30°C und tropft innerhalb 10 Minuten 20 ml einer standardisierten Jones-Lösung zu. Man rührt noch 1,5 Stunden bei -20°C nach, zersetzt überschüssiges Oxidationsmittel durch Zugabe von 25 ml Isopropanol, läßt auf 20°C erwärmen, versetzt mit 300 ml Wasser und extrahiert mehrfach mit insgesamt 600 ml Diethylether. Die vereinigten organischen Extrakte wäscht man mit Wasser und gesättigter Natriumchloridlösung bis zur Neutralreaktion, trocknet über Magnesiumsulfat und reinigt den nach Filtration und Lösungsmittelabzug erhaltenen Rückstand durch Chromatographie an ca. 800 ml feinem Kieselgel unter Verwendung eines n-Hexan/Ethylacetat-Gemisches. Isoliert werden neben 11,3 g (23,9 mmol, 64%) (1S,5R,6S,7R)-7-Benzoyloxy-6-[(E)-(4S)-2-brom-3-oxo-4-methyl-1-nonen-6-inyl]-2-oxa-bicyclo [3.3.0]octan-3-on 3,59 g (7,58 mmol, 26%) (1S,5R,6R,7R)-7-Benzoyloxy-6-[(Z)-(4S)-1-oxo-2-brom-4-methyl-non-2-en-6-inyl]-2-oxabicyclo[3.3.0]octan-3-on. Letztere Verbindung löst man in 45 ml wasserfreiem Methanol, kühlt unter einer Atmosphäre aus trockenem Argon auf -45°C, versetzt portionsweise mit insgesamt 520 mg Natriumborhydrid und rührt 30 Minuten bei -45°C nach. Überschüssiges Reduktionsmittel zersetzt man durch Zugabe von 0,8 ml Aceton, läßt auf 20°C erwärmen, versetzt mit Wasser und extrahiert mehrfach mit Diethylether. Die vereinigten organischen Extrakte wäscht man mit Wasser und gesättigter Natriumchloridlösung bis zur Neutralreaktion, trocknet über Magnesiumsulfat und reinigt den nach Filtration und Lösungsmittelabzug erhaltenen Rückstand durch Chromatographie an ca. 200 ml feinem Kieselgel unter Verwendung eines Dichlormethan/Ethylether-Gemisches. Isoliert werden 1,79 g (3,76 mmol, 50%) des unpolaren Alkohols sowie 528 mg (1,11 mmol, 15%) des polaren Alkohols.

## Patentansprüche

1. Verfahren zur Herstellung von Prostaglandinvorstufen der Formel I 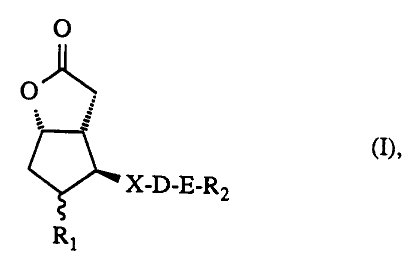 sowie deren Enantiomere
worin
X A-W oder W-A,
A eine ― C ≡ C ― Gruppe,
W eine Hydroxymethylengruppe, in der die OH-Gruppe durch Veretherung oder Veresterung funktionell abgewandelt sein kann,
D eine geradkettige oder verzweigtkettige Alkylengruppe mit jeweils 2-5 C-Atomen oder eine 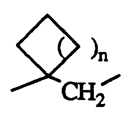 -Gruppe,
n 1 bis 3,
E eine ― C ≡ C ― Gruppe oder eine -CR₃=CR₄-Gruppe mit R₃ und R₄ jeweils in der Bedeutung eines Wasserstoffatomes oder einer C₁-C₄-Alkylgruppe,
R₁ ein Wasserstoffatom oder eine Hydroxygruppe, die wie in W funktionell abgewandelt sein kann,
R₂ eine geradkettige oder verzweigtkettige Alkylgruppe mit 1-7 C-Atomen ist, dadurch gekennzeichnet, daß man Vinylbromide der Formel II 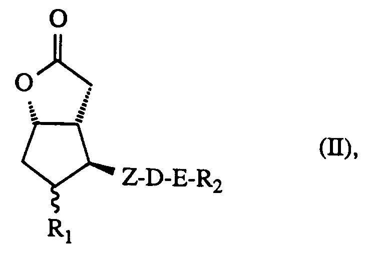
worin Z die Gruppe 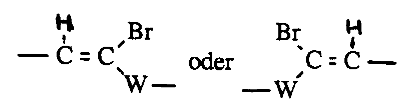 bedeutet und R₁, W, D, E und R₂ die oben angegebenen Bedeutungen haben und Hydroxygruppen in R₁ und W ungeschützt oder geschützt sein können durch einen gegebenenfalls substituierten Benzoylrest, eine C₁-C₆-Alkanoylgruppe, einen Tetrahydropyranylrest, einen Tetrahydrofuranylrest, eine Trialkylsilylgruppe oder eine Diphenylalkylsilylgruppe jeweils mit Alkyl in der Bedeutung eines C₁-C₄-Alkyls, mit wasserfreiem Cäsiumacetat in Gegenwart von 18-Krone-6 umsetzt.

## Claims

1. Process for the preparation of prostaglandin precursors of formula I 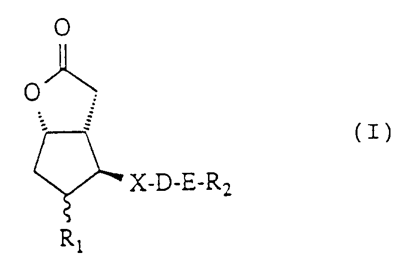 and their enantiomers, in which
X is A-W or W-A,
A is a group -C≡C-,
W is a hydroxymethylene group in which the OH group can be functionally modified by etherification or esterification,
D is a straight-chained or branched-chained alkylene group having in each case from 2 to 5 carbon atoms, or a group 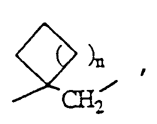
n is 1 to 3,
E is a group -C≡C- or a group -CR₃=CR₄- wherein R₃ and R₄ each represent a hydrogen atom or a C₁-C₄alkyl group,
R₁ is a hydrogen atom or a hydroxy group that can be functionally modified as in W,
R₂ is a straight-chained or branched-chained alkyl group having from 1 to 7 carbon atoms,
characterised in that vinyl bromides of formula II 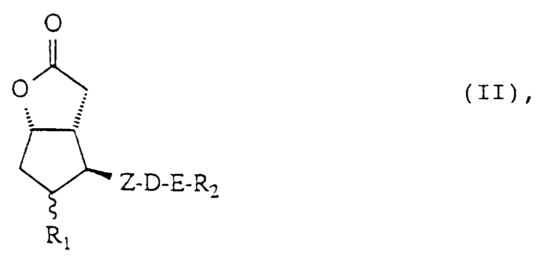
in which Z represents the group 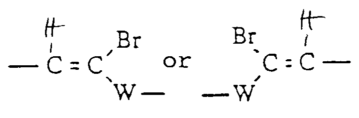 and R₁, W, D, E and R₂ have the meanings given above and hydroxy groups in R₁ and W are unprotected or protected by an optionally substituted benzoyl radical, a C₁-C₆-alkanoyl group, a tetrahydropyranyl radical, a tetrahydrofuranyl radical, a trialkylsilyl group or a diphenylalkylsilyl group, alkyl being in each case a C₁-C₄alkyl, are reacted with anhydrous caesium acetate in the presence of 18-crown-6.

## Revendications

1. Procédé pour la préparation de précurseurs de prostaglandines de formule I 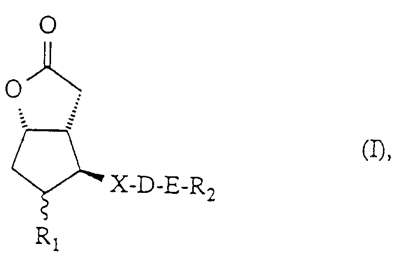 ainsi que de leurs énantiomères
où
X représente A-W ou W-A,
A représente un groupe --C ≡ C--,
W représente un groupe hydroxyméthylène, dans lequel le groupe OH peut être fonctionnellement transformé par éthérification ou estérification,
D représente un groupe alkylène linéaire ou ramifié chaque fois comportant de 2 à 5 atomes de carbone ou un groupe 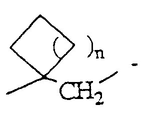
n vaut 1 à 3,
E représente un groupe --C ≡ C -- ou un groupe -CR₃=CR₄ avec R₃ et R₄ ayant chaque fois la signification d'un atome d'hydrogène ou d'un groupe alkyle en C₁-C₄,
R₁ représente un atome d'hydrogène ou un groupe hydroxy qui peut être fonctionnellement transformé comme dans W,
R₂ représente un groupe alkyle linéaire ou ramifié comportant de 1 à 7 atomes de carbone, caractérisé en ce qu'on fait réagir des bromures de vinyle de formule II 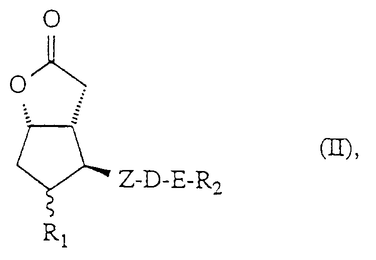
dans laquelle Z représente le groupe 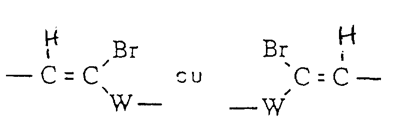 et R₁, W, D, E et R₂ ont les significations données ci-dessus, et les groupes hydroxy dans R₁ et W non protégé ou protégé par un radical benzoyle éventuellement substitué, représente un groupe alcanoyle en C₁-C₆, un radical tétrahydropyranyle, un radical tétrahydrofuranyle, un groupe trialkylsilyle ou un groupe diphénylalkylsilyle avec l'alkyle chaque fois ayant la signification d'un alkyle en C₁-C₄, avec l'acétate de césium anhydre en présence de 18-couronne-6.
